# EUROPEAN PATENT APPLICATION

(11) **EP 3 791 753 A1**
(43) Date of publication of application: **17.03.2021**
(21) Application number: 20000152.7
(22) Date of filing: 03.09.2019
(51) Int. Cl.: A47C 7/72, A47C 31/12, A61B 5/00

(54) **ERGONOMIC CHAIR WITH EMBEDDED PRESSURE AND DISTANCE SENSORS FOR DETECTION AND PREVENTION OF INCORRECT POSTURE**

(62) Divisional of application: 19000394.7
(71) Applicant: "Cluster Mobilier Transilvan" Association, Cluj County (RO)
(72) Inventor: Levente, Denes, Harghita County (RO); Bogdan, Movileanu, Brasov County (RO); Flutur, George, Iasi County (RO); Cosovanu, Daniel, Suceava County (RO); Ionut-Danut, Danci, Satu Mare County (RO); Levente, Nemes, Cluj County (RO)

(57) **Abstract**

An embedded system in an ergonomic chair for training a user to maintain correct posture while seated, containing a microcontroller with processing capabilities and access to the Internet which is capable of collecting data from each of the sensors from the chair with this embedded system. The system also includes a software application divided into embedded application and web application. The embedded application is a component that communicates with the integrated sensors, analyzes the data, and then they are sent to a database, and the web application is responsible for analyzing and displaying data to the user in a suggestive manner.

## Description

### Field of the Invention

This invention is generally related to the field of ergonomic devices and methods useful for improving posture. More specifically, this invention is related to apparatus and methods for sensing when a user is seated in a chair in an ergonomically correct position with proper posture, monitoring the user's posture over time and reporting the results of the monitoring to the user.

More particularly, it also incorporates aspects of various engineering subsets: mechanics and mechatronics for chair design and building, electrical for sensor design and battery circuit design and implementation and software for the microcontroller and the web platform.

### Description of Prior Art

The inclusion of Internet of Things (IoT) in medical applications is largely due to the fact that IoT systems offer many features used in remote patient.

Nowadays, even if office work does not involve physical work, sitting is a risk factor in the occurrence of one of the most widespread chronic diseases / spinal disorders, leading to lower back pain and movement dysfunction.

The lower back pains appear at nearly every adult on the globe. Taking a more in depth look at this issue, it has been discovered that over 20% of all Americans consider the source of their back pains to be a bad position while sitting at work, while 46% of the French believe that a bad posture is the root of their problem. The back pains impact their productivity at work as well. The low-back pains costing employers $51,400 annually per 100 employees in lost productivity and medical treatments.

On average, in Europe more than 50% of the employees use the computer at least half a day. This leads to increased physical inactivity and only repetitive movements in the shoulder, arm and wrist. All these increase the number of diseases related to the skeletal muscle system.

There is, therefore, a need for developing a solution for posture correction that shows a full-analysis of the user's back, indicating the areas with problems and also their evolution in time.

Considering the prior art that we are aware of (US20110275939A1), the novelty from the technical point of view is that the chair embedded newly developed sensors which measure not only the body pressure (used almost exclusively in the newly developed smart chairs) but also the distance between the user's body and sensors too and also rotation of the user's body. The configuration of the six sensors, four placed in the back and two in the seat, can detect all body postures so the chair warns the user about the incorrect body position. For detection is used the combination of the distances and pressures as the key parameters of the system. The pressures are measured if the user's body touches the sensors. This combined information (distance + pressure) is more accurate and offers wider opportunities to detect the exact body postures, to collect and analyze the sitting behavior of the users instantaneously or for a determined period of time. From data acquisition and user-product interaction point of view, the novelty consists in the data representation and how the system monitors, reveals and warns the users about their postures, furthermore how the system offers statistics about a certain period of use. In the case of other similar solutions, the chair offers a feedback about the user's postures, but in this case the feedback is given by a developed application. By this solution the chair user is warned visually about the incorrect postures. The visual warning can be placed on the user's computer screen and/or on cellphone displays. The seating-sensing-data processing-evaluating loop is closed by the user notification about the current posture and in the case of an uncomfortable position the user can correct his/her body posture.

### Summary of the Invention

The ergonomic chair provides embedded sensors in the chair. The sensors in the seat are used to determine the position of the legs, and those in the back for determining the posture, by measuring the distance from the backrest to the six areas of the back.

The seat sensors, by distributing the load consisting of the weight of the human body, identify the pressure with which they act on the areas of interest. As for the sensors in the back, these are used to determine the evolution of the orientation curve of the spine and at the same time by analyzing the distribution of the back plane in space to detect the shape of the posture.

By receiving and analyzing the electrical signal received from the six sensors, the electronic module communicates with the Internet network at the workplace.

The electrical signal is taken and transformed into an electromagnetic signal which is received by the Internet network used. The entire system is powered by the battery encapsulated by the electronic module provided with a charging port.

The software application is divided into two major categories: web application and embedded application. The embedded application is a component that communicates with the integrated sensors, analyzes the data, and then they are sent to the Google Firebase database. The web application is responsible for analyzing and displaying data to the user from Google Firebase in a suggestive manner.

The ergonomic chair of the present invention can assist in creating a clear path to help people learn and maintain healthier sitting habits and, thus, reduce risk of future injury. In businesses environments, this solution can help employees to create better postural habits and reduce a vast number of medical conditions. This would help people live healthier, more enjoyable lives, especially by allowing employees to be more productive and by saving the government, employers and individuals large amounts of money each year.

### Brief description of the drawings

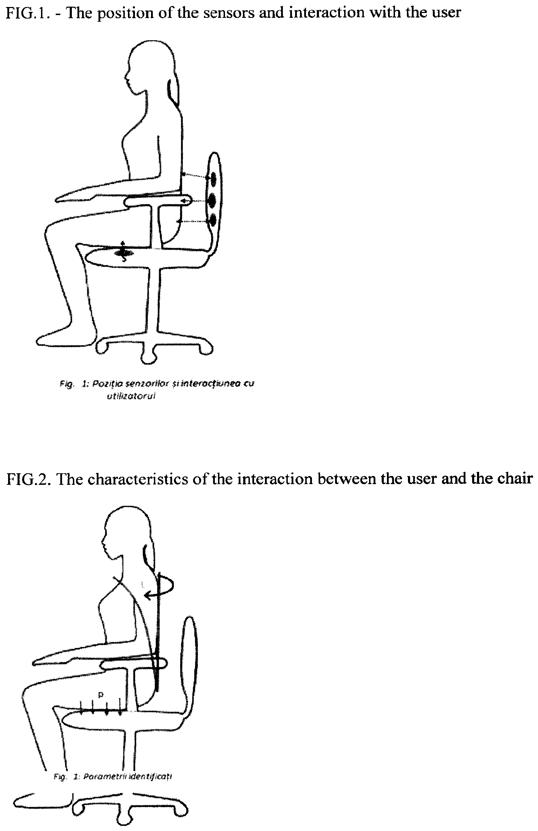

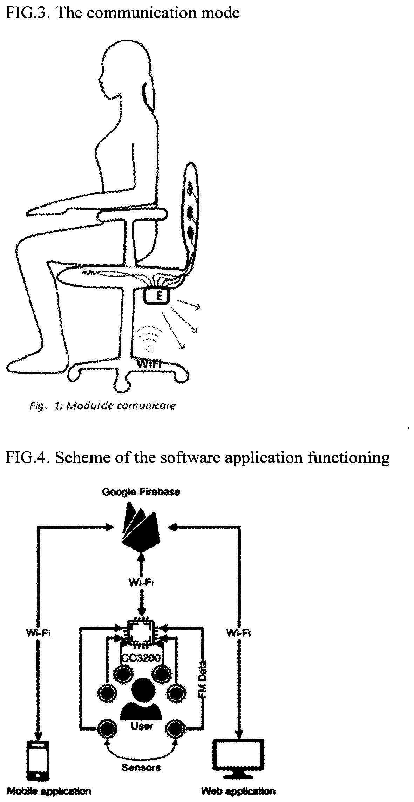

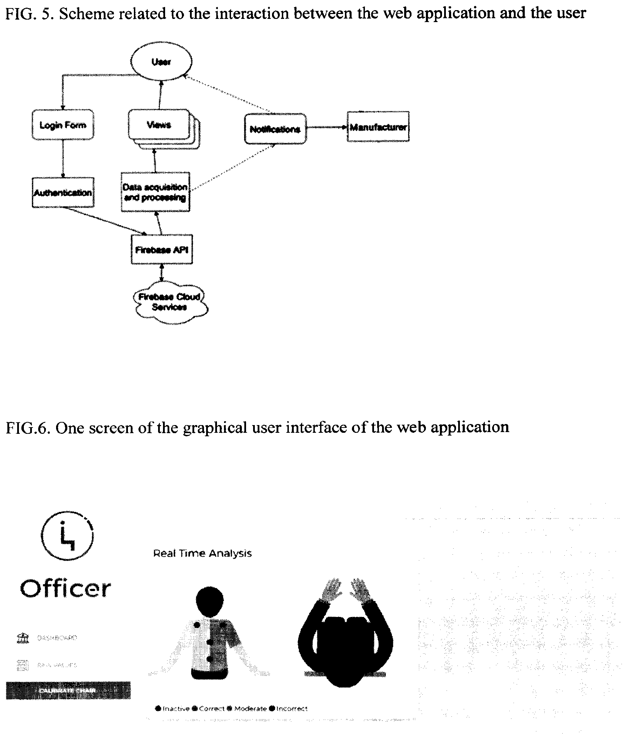

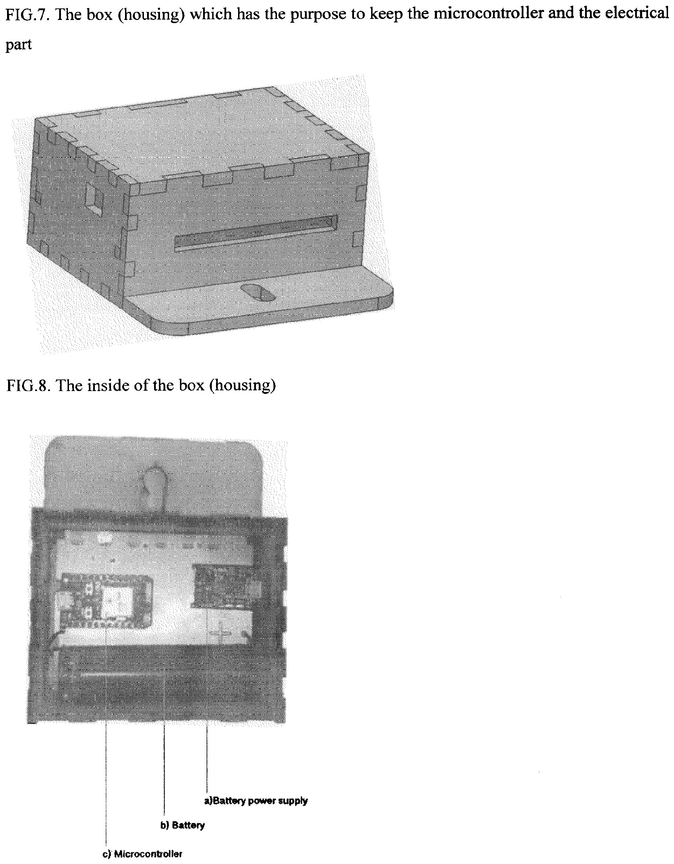

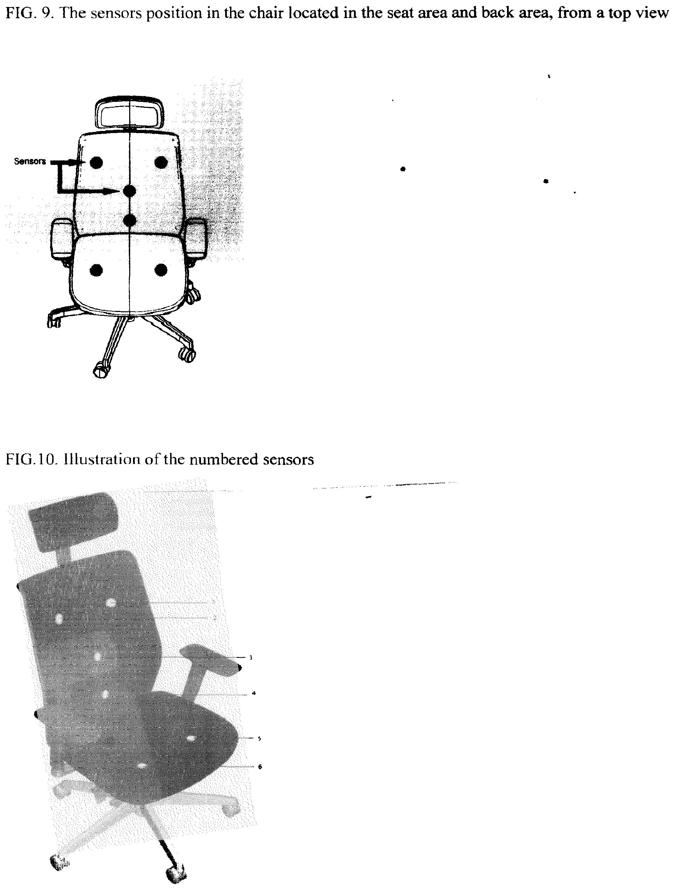

The attached figures and drawings show, as illustrative examples, embodiments of the present invention:
o FIG. 1 provides an illustration of the position of the sensors and interaction with the user
o FIG. 2 illustrates the characteristics of the interaction between the user and the chair, which are the identified parameters
o FIG. 3 provides an illustration of the communication solution used by the chair and the communication mode
∘ FIG. 4 is a scheme of the software application functioning
∘ FIG. 5 provides an illustration of a scheme related to the interaction between the web application and the user
∘ FIG. 6 provides an illustration of one screen of the graphical user interface of the web application
∘ FIG. 7 provides an illustration of the box or housing which has the purpose to to keep the microcontroller and the electrical part
∘ FIG. 8 provides an illustration of the inside of the aforementioned box
∘ FIG. 9 provides an illustration of the sensors position in the chair located in the seat area and back area, top view
∘ FIG. 10 provides an illustration of the numbered sensors

### Detailed description of the invention

An embodiment of the present invention includes, as illustrated in FIG. 1, the ergonomic chair with embedded sensors. They are placed in the seat (two sensors) and in the backrest (four sensors) with well-established positions in order to allow interaction with some essential areas of the human body.

The sensors in the seat are used to determine the position of the legs, and those in the back for determining the posture, by measuring the distance from the backrest to the six areas of the back, these being:
- left scapula
- right scapula
- central spinal
- lower right lumbar area
- lower left lumbar area.

The seat sensors, by distributing the load consisting of the weight of the human body, identify the pressure (p) with which they act on the areas of interest, as illustrated in FIG.2. The sensors located in the backrest are used as proximity and rotation sensors, so they determine the evolution of the orientation curve of the spine (L) and the rotation of the upper body and at the same time they analyze the distribution of the back plane in space to detect the shape of the posture.

Following data collection, by receiving and analyzing the electrical signal received from the six sensors, the electronic module (E), as illustrated in FIG.3, communicates with the Internet network at the workplace. Within the electronic module (E) attached to the seat, there are integrated:
- the microcontroller
- the dedicated Printed Circuit Boards (PCB)
- the battery
- the port of loading
- the housing

The sensors are connected via the dedicated PCB, using the jacks, with the microcontroller. The electrical signal is transformed into an electromagnetic signal which is then received by the Internet network used. The entire system is powered by the battery encapsulated by the electronic module. At the same time, a charging port is used to charge the battery, making the connection through the dedicated PCB. The housing which is used for the protection of the module is made of wood. The rest of the functionalities of the ergonomic type of office are the usual ones, which can be identified in a normal chair.

The software application is divided into two major categories: web application and embedded application. The embedded application is a component that communicates with the integrated sensors, analyzes the data, and then they are sent to the Google Firebase database. The web application is responsible for analyzing and displaying data to the user from Google Firebase in a suggestive manner. From an architectural point of view, the system is presented in the FIG. 4.

Once the sensor data, measured using the microcontroller, reaches the database the web application will access this data and display it to the logged in user. Also, the entire web application is responsible for notifying the user when his position is wrong or when he has spent too much time on the chair. The interaction between the web application and the user is illustrated in FIG. 5.

In order to be able to display in real time the position where the user is located, two images were used in which the measured points were highlighted. Each monitored area will change the color of the points in the figure according to correctness.

Because the measured data must be reported at a correct position, the user will be able to calibrate his seat relative to this position. In order to do this, he may press the "Calibrate Chair" button in the menu on the left. The graphical interface of the web application is illustrated in FIG.6.

The embedded application is implemented and runs on a particle photon microcontroller with integrated Wi-Fi mode. Through this application, the microcontroller measures the data that each sensor takes for 100 milliseconds, and after all the six sensors have been read, the values will be sent to the database every second. Moreover, every hour the average of the values on the sensors is calculated so that after this information is sent to the database, the user will know his position in each hour.

The sensors 5 and 6 are inserted into the back and have a special housing, they are placed at 12 cm from the side edges and at 16 cm from the front edge, as illustrated in FIG. 10.

The sensors 1 and 2 are positioned 7 cm from the edge horizontally, and 9 cm from the edge vertically. The sensors 3 and 4 are centered on the horizontal and distance compared to the upper edge is 30 cm, respectively 42 cm, see FIG 9 and FIG 10. These sensors are placed in special pockets, square shaped, with a side of 4.5 cm which should be sewn on the middle layer of the mesh.

A further embodiment is the sensor operating mode through distance measurement and pressure measurement. The sensors are based on the capacitive principle. That is why the user's interaction with such a sensor can provide more information than an ordinary sensor. There are two data profiles for the user: the distance between the user's area closest to the sensor and the sensor can be estimated compared to a distance already measured. Moreover, there is also pressure measurement, when the distance between the user and the sensor becomes null, the intensity with which the sensor is pressed can be estimated precisely also in comparison with previous results.

A further embodiment is the housing (a box) that has the role of keeping the electrical and control elements in a way that avoids any danger and interaction, see FIG. 7, FIG. 8. The housing is not meant to interact with the user, but to keep the microcontroller and the electrical part safe. There is a set of cables attached to the housing. These cables connect the sensors and the microcontroller inside the box. For this purpose, the box was designed with slots to meet the need to pull cables between components. These cables have paths from the sensors on the side, through the outside, down to the box. The cables are 60 cm in size and are directly plugged. Inside the box there are:
- The battery power supply circuit
- The Microcontroller
- The Battery

The battery supplies the circuit. Its function is to provide the necessary working power to the circuit. The microcontroller is the brain of the process. It collects data from sensors, sends them for processing. It has its own processing capabilities and even Internet access.

The battery power circuit is a support element. Its main function is to ensure optimal battery parameters.

The invention is not limited to the details of the foregoing illustrated embodiments and the present invention may be embodied in other specific forms without departing from the spirit or essential attributes thereof. The present embodiments are therefore to be considered in all respects as illustrative-and not restrictive-the scope of the invention being indicated by the appended claims rather than by the foregoing description, and all changes which come within the meaning and range of equivalency of the claims are therefore intended to be embraced therein. As stated above, the novelty elements are given by the introduction of the sensors in the chair being embedded, the way of placing them and what they send as a signal to the software, as well as the interpretation of the data for the user of the chair along with the software application.

It should be emphasized that the described embodiments are possible examples of implementations, merely set forth for a clear understanding of the principles. Variations and modifications may be made to the described embodiment(s) without substantially departing from the spirit and principles of the present teaching. All such modifications are intended to be included herein within the scope of this disclosure and the present invention and protected by the following claims. For example, steps associated with the processes described herein can be performed in any order, unless otherwise specified or dictated by the steps themselves. The present disclosure is intended to embrace all such alternatives, modifications and variances that fall within the scope of the appended claims.

## Claims

1. An embedded system in the chair for training a user to maintain correct posture while seated in a chair with a seat portion and a back portion, with one or more pressure and distance sensors, a microcontroller for collecting data from the one or more pressure and distance sensors, operably connected through a software application that communicates with the sensors, analyzes the data, sents them to a database, and also analyzes and displays data to the user.

2. The system of claim 1 further comprising the possibility to send data after users' approval to chair producers in order to facilitate the future chair developments or to health organizations to support a proper diagnosis in case of user's back pain problems
